# EUROPÄISCHE PATENTANMELDUNG

(11) **EP 1 647 302 A1**
(43) Veröffentlichungstag der Anmeldung: **19.04.2006**
(21) Anmeldenummer: 05021695.1
(22) Anmeldetag: 05.10.2005
(51) Int. Cl.: A61N 1/365, A61N 1/375

(54) **Elektromedizinisches Implantat**

(30) Priorität: 12.10.2004 DE 102004049778; 06.12.2004 DE 102004059096; 20.04.2005 DE 102005018128
(71) Anmelder: BIOTRONIK CRM Patent AG, 6340 Baar (CH)
(72) Erfinder: Drews, Jürgen, Dr., 01796 Pirna (DE); Hickmann, Steffen, 01809 Heidenau (DE); Fehrmann, Gerd, 01796 Pirna (DE); Neumann, Wiebke, 12209 Berlin (DE); Staub, Roland, 01819 Borggiesshübel (DE); Uhrland, Werner, 12355 Berlin (DE)
(74) Vertreter: Lindner-Vogt, Karin L.

(57) **Zusammenfassung**

Elektromedizinisches Implantat (20) bestehend aus einem nach.außen hermetisch abgeschlossenen Gehäuse (21), einer Energieversorgungseinheit (10) mit einer ersten Schale (11) bestehend aus einer 1. elektrisch leitfähigen Hauptfläche (11.1) und einer 1. Seitenwand (11.2), und einer zweiten Schale (12) bestehend aus einer 2. Hauptfläche (12.1) und einer 2. Seitenwand (12.2), die in das nach außen hermetisch abgeschlossene Gehäuse (21) eingebettet ist, einer mit der Energieversorgungseinheit (10) elektrisch verbundenen elektrischen Steuerungseinheit (33), einem Header zur Kontaktierung von Elektrodenleitungen, Durchführungen zum Abführen von therapeutischen Impulsen oder Impulsfolgen aus dem nach außen hermetisch abgeschlossenen Gehäuse. Die elektrische Steuerungseinheit (33) ist dabei über die 1. Hauptfläche (11.1) der Energieversorgungseinheit (10) zweipolig elektrisch verbunden, die 2. Hauptfläche (12.1) der Energieversorgungseinheit (10) hat mindestens die 0,7-fache Fläche der Grundfläche des nach außen hermetisch abgeschlossenen Gehäuses (21) des elektromedizinischen Implantats (20), die Höhe der Energieversorgungseinheit (10) beträgt höchstens die 0,5-fache Höhe des nach außen hermetisch abgeschlossenen Gehäuses (21) des elektromedizinischen Implantats (20).

## Beschreibung

Die vorliegende Erfindung betrifft ein elektromedizinisches Implantat mit einer Energieversorgungseinheit zur einfachen und kostengünstigen Herstellung.

Die interkardiale Herztherapie hat sich mittlerweile zu einem millionenfach bewährten Standardverfahren entwickelt. Dabei wird ein elektromedizinisches lmplantat in eine Hauttasche eines zu therapierenden Patienten implantiert und zum Beispiel dauerhaft über einer Elektrodenleitung elektrisch mit dem Herzen verbunden. Bei einem solchen elektromedizinischen Implantat handelt es sich um einen Herzschrittmacher oder einen implantierbaren Defibrillatoren, Medikamentenpumpen, Neurostimulatoren oder jedem anderen, elektrische Energie aussendenden Gerät, das in einen menschlichen oder tierischen Körper implantiert wird.

Die große Herausforderung bei einem elektromedizinischen Implantat ist die optimale Platz sparende Raumausnutzung im begrenzten geringen Volumen des Gehäuses eines solchen Implantats. Bis jetzt wird ein elektromedizinisches Implantat in einem "Side-by-Side-Aufbau" hergestellt. Das bedeutet, dass die einzelnen Komponenten eines elektromedizinischen Implantats auf der Grundfläche nebeneinander angeordnet sind. So liegt zum Beispiel die Energieversorgungseinheit auf der Grundfläche eines elektromedizinischen Implantats neben der elektrischen Steuerungseinheit eines elektromedizinischen Implantats. Sehr nachteilig an dieser Bauweise ist, dass eine hohe manuelle Tätigkeit erforderlich ist, um ein in "Side-by-Side-Bauweise" aufgebautes elektromedizinischen Implantat zu fertigen und dabei die genannten Anforderungen an die Raumausnutzung sicherzusteLLen.

Deshalb ist man schnell dazu übergegangen, die Herstellung derartiger Geräte zu vereinfachen und zu automatisieren. Dies geht mit einer Vereinfachung und Standardisierung des Aufbaus eines Schrittmachers einher. Von dieser Vereinfachung ist natürlich auch die Produktion betroffen, was bedeutet, dass ein elektromedizinisches Implantat mit wesentlich geringeren Kosten hergestellt werden kann. Dabei hat sich der "Bottom-up-Aufbau" als sehr vorteilhaft erwiesen. "Bottom-up" bedeutet, dass die Komponenten eines elektromedizinischen Implantats übereinander aufgebaut sind. Die großen Komponenten werden dabei als Erstes eingebaut. Auf diesen kommen die leichteren kleineren Komponenten zu liegen. Die größte Komponente bleibt nach wie vor die Energieversorgungseinheit, da eine sichere und möglichst lang anhaltende elektrische Versorgung von elementarer Wichtigkeit für ein elektromedizinisches Implantat ist. Für diese Anforderung spricht ein größtmöglicher Komfort, der dem Patienten geboten werden muss. Dazu gehört auch, die Anzahl von Nachsorgeuntersuchungen zu minimieren. Deshalb werden diese Energieversorgungseinheiten so ausgelegt, dass eine möglichst lange Lebensdauer ermöglicht wird. Leider hängt bei Energieversorgungseinheiten die Kapazität vom Volumen ab, weshalb die Energieversorgungseinheit über eine noch nicht absehbare Zeit die größte Komponente eines elektromedizinischen Implantats bleibt und deshalb auch die unterste Komponente bei der "Bottom-up-Bauweise bleibt. Unter Energieversorgungseinheit sollen im Übrigen alle Batterien, Akkumulatoren oder andere bekannte Energieerzeugungseinrichtungen verstanden werden.

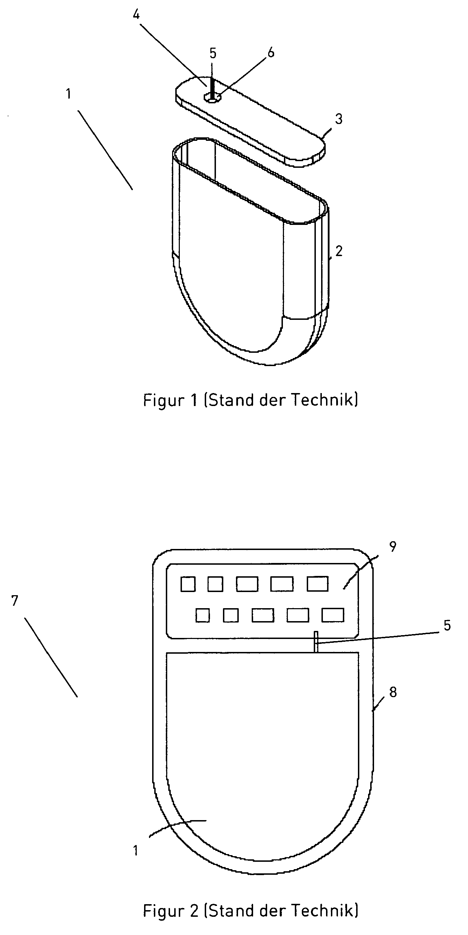

Ein Beispiel einer Energieversorgungseinheit für den oben genannten "Side-by-Side-Aufbau" ist eine hermetisch geschlossene Batterie 1 wie in Figur 1 dargestellt. Die halboval geformte Batterie besteht aus einem Gehäuse 2 und einem Deckel 3. Weiterhin ist hier die Durchführung 4 auf dem Deckel 2 zu sehen, welche von außen sichtbar aus einem Durchführungspin 5 und einer Buchse 6 besteht. In der Regel werden hierzu Glas-Metall-Durchführungen verwendet, deren Metallbuchse in eine Bohrung des Deckels eingeschweißt wird. Wenn die Batterie mit einem flüssigen, gelförmigen oder polymeren Elektrolyten befüllt wird, ist im Deckel 3 der Batterie 1 gewöhnlich eine Öffnung vorgesehen, welche nach dem Füllen hermetisch verschlossen wird. Hierzu wird in der Regel ein Verschlussstück eingeschweißt oder eingenietet.

Figur 2 zeigt ebenfalls einen aus dem Stand der Technik bekannten Aufbau eines elektromedizinischen Implantats 7. Die Energieversorgungseinheit 1 ist passförmig in das hermetisch dichte Gehäuse 8 des elektromedizinischen Implantats 7 eingebettet. Im hermetisch dichten Gehäuse 8 des elektromedizinischen Implantats 7 wird die elektronische Steuerungseinheit 9 oberhalb der Energieversorgungseinheit 1 angeordnet und elektrisch über den Durchführungspin 5 mit der Energieversorgungseinheit 1 verbunden. Nachteil einer solchen Energieversorgungseinheit besteht darin, dass durch die Lage der Durchführung an der Flachseite einer Energieversorgungseinheit keine kostengünstige "Bottom-up-Bauweise" möglich ist. Ein Beispiel für eine solche Energieversorgungseinheit ist in US 4,830,940 dargestellt.

Im Patent US 6,613,474 B2 wird eine Flachbatterie beschrieben, die auf dem Zusammenfügen zweier passgenauer metallener Gehäuse-Halbschalen beruht. Beide Gehäuseteile sind passgenau zusammengefügt, um so ein hermetisches Verschweißen zu erleichtern. Nachteil dieser Erfindung ist ebenfalls, dass durch die Lage der Durchführung an einer flachen Seite ein kostengünstiger "Bottom-up-Aufbau" nicht möglich ist.

WO 02/32503 A1 beschreibt ein elektromedizinisches Implantat mit einer Batterie. In der genannten Veröffentlichung wird beschrieben, dass das implantierbare Gerät aus einem Batterie- und einem Elektronikteil derart aufgebaut ist, dass mindestens eine Stirnseite der Energieversorgungseinheit die Außenseite des elektromedizinischen Implantats bildet. Dies stellt quasi eine "Bottom-up-Bauweise" dar, da die große Komponente einfach an die kleineren Komponenten angebaut wird. Der große Nachteil hierbei ist jedoch, dass ein Teil des Gehäuses der Energieversorgungseinheit gleichzeitig das Außengehäuses eines elektromedizinischen Implantats darstellt. Bei einem Leck im Gehäuse der Batterieeinheit können bei einem Patienten schwerwiegende toxische Folgen eintreten.

Ein Beispiel für eine "Bottom-up-Bauweise" wird in der EP 1 407 801 A2 dargestellt. Die Steuerungseinheit eines elektromedizinischen Implantats ist auf einer Energieversorgungseinheit aufgebaut, welche durch eine Flachseite, eine Unterseite und eine umlaufende Schmalseite gekennzeichnet ist. Hierdurch wird es möglich, das implantierbare Gerät durch eine einfache "Bottom-up-Bauweise" herzustellen, da die Steuerungseinheit des implantierbaren Geräts auf der Flachseite der Energieversorgungseinheit aufgebaut werden kann.

Vorteil dieser Methode ist eine optimale Volumenausnutzung, welches durch das Gehäuse des elektromedizinischen Implantats begrenzt ist.

Die Aufgabe der Erfindung ist es, die oben genannten Nachteile zu vermeiden und ein elektromedizinisches Implantat mit einer Energieversorgungseinheit zur Verfügung zu stellen, welches mit der kostengünstigen "Bottom-up-Bauweise" hergestellt werden kann. Das elektromedizinische Implantat besteht aus einem nach außen hermetisch abgeschlossenen Gehäuse und einer vorteilhaften Energieversorgungseinheit aus einer ersten Schale mit einer 1. elektrisch leitfähigen Hauptfläche und einer 1. Seitenwand, und einer zweiten Schale bestehend aus einer 2. Hauptfläche und einer 2. Seitenwand. Die Energieversorgungseinheit wird in das nach außen hermetisch abgeschlossene Gehäuse eingebettet. Mit der Energieversorgungseinheit wird eine elektrische Steuerungseinheit elektrisch verbunden. Als besonders vorteilhaft hat sich herausgestellt, die elektrische Steuerungseinheit über die 1. Hauptfläche der Energieversorgungseinheit zweipolig elektrisch zu verbinden und die Dimensionen der Grundfläche der Energieversorgungseinheit sowohl in Form als auch in Größe der Grundfläche des elektromedizinischen Implantats anzupassen. Dies ermöglicht eine einfache Positionierung der Energieversorgungseinheit im Gehäuse des elektromedizinischen Implantats und verhindert versehentlichen fehlerhaften oder falschen Einbau der Energieversorgungseinheit in das elektromedizinische Implantat.

Die flache 1. Hauptfläche ermöglicht die direkte Befestigung der Steuerungseinheit. Die 1. Hauptfläche ist so ausgeführt, dass sie genügend Raum zur Montage der Steuerungseinheit zur Verfügung stellt. Die 1. Hauptfläche ist mit einer Glas-Metall-Durchführung, einer Befüllöffnung und Kontaktelementen versehen, die eine "bottom-up-Montage" ermöglichen und so angeordnet, dass die direkte elektrische Kontaktierung der elektrischen Steuerungseinheit ermöglicht wird. Die Glas-Metall-Durchführung und die Befüllöffnung sind in der 1. Hauptfläche versenkt eingebaut, wodurch eine absolut flache Montage der Steuerungseinheit ermöglicht wird. In der Energieversorgungseinheit kommt ein spezielles Druckstück zum Einsatz, welches zur Stabilität des inneren Aufbaus der Energieversorgungseinheit beiträgt. In der Energieversorgungseinheit kommt ein spezieller Haltering zum Einsatz, der die Verwendung komplexer Geometrien erheblich vereinfacht und gleichzeitig zur Stabilität des inneren Aufbaus der Energieversorgungseinheit beiträgt. In der Energieversorgungseinheit kommt ein spezielles Ableitungsblech zum Einsatz, welches eine elektrisch leitfähige Verbindung zwischen dem Pin der Glas-Metall-Durchführung und dem Ableitungsgitter der Elektrode herstellt. Dieses Ableitungsblech vereinfacht die Herstellung der Energieversorgungseinheit und vereinfacht die Kontaktierung von Elektroden mit komplexer Geometrie. Die Schwellung der Energieversorgungseinheit kann durch die Verwendung der 1. Hauptfläche mit abgewinkelter Geometrie verringert werden und die mechanische Stabilität erhöht werden.

Es zeigen:
- Figur 1:: eine Energieversorgungseinheit aus dem Stand der Technik
- Figur 2:: eine eingebaute Energieversorgungseinheit in ein elektromedizinisches Implantat aus dem Stand der Technik
- Figur 3:: eine Energieversorgungseinheit eingebaut in ein elektromedizinisches Implantat im Teilschnitt in der Aufsicht
- Figur 4:: eine Energieversorgungseinheit im Explosionsmodell
- Figur 5A bis Figur 5D:: Ausführungsbeispiele zur Geometrie einer Energieversorgungseinheit in der Aufsicht
- Figur 6:: Aufbau der Seitenwände beim hermetischen Schweißen
- Figur 7A und Figur 7B:: Ausführungsbeispiele zur Stabilität und Verhinderung der Schwellung einer Energieversorgungseinheit in der Seitenansicht
- Figur 8:: montierte Kontaktelemente auf der 1. Hauptfläche einer Energieversorgungseinheit
- Figur 9:: eine zweigeteilte 1. Hauptfläche zur Kontaktierung zweier Pole
- Figur 10:: eine Glas-Metall-Durchführung in einem Schnittbild
- Figur 11:: Innenaufbau einer Energieversorgungseinheit mit Spannrahmen
- Figur 12:: Innenaufbau einer Energieversorgungseinheit mit Glas-Metall-Durchführung und Befüllöffnung im Teilschnitt
- Figur 13:: ein Kontaktblech
- Figur 14A und 14B:: die Montagemöglichkeiten eines Kontaktbleches
- Figur 15A bis Figur 15C:: die Montage des Kontaktblechs

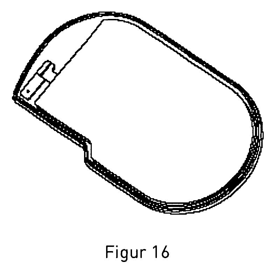

- Figur 16:: eine komplett_montierte Energieversorgungseinheit

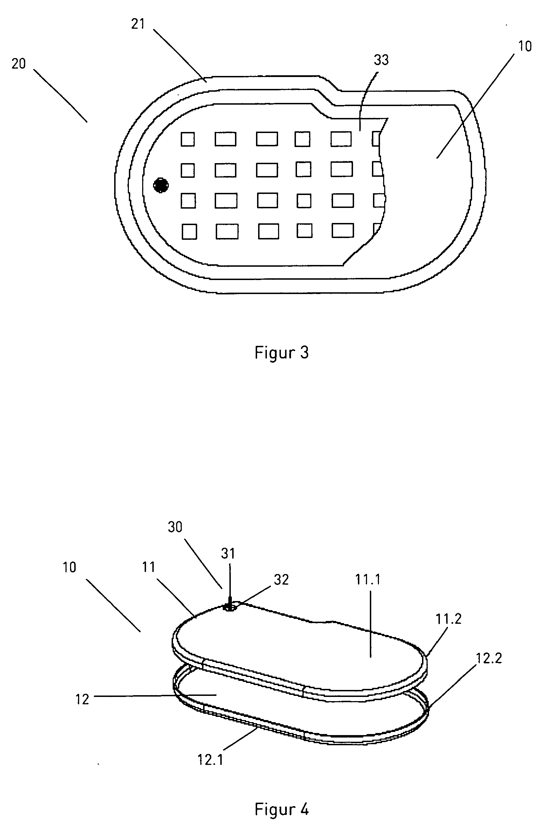

Figur 3 zeigt den montierten Zustand eines elektromedizinische Implantats 20. Zu sehen ist das nach außen hermetisch abgeschlossene Gehäuse 21, die Energieversorgungseinheit 10 und die auf der 1. Hauptfläche 11.1 der Energieversorgungseinheit montierte elektrische Steuerungseinheit 33. Die 1. Hauptfläche 11.1 ist so ausgeführt, dass sie die elektrische Steuerungseinheit 33 des implantierbaren Geräts 10 aufnehmen kann. Das Größenverhältnis zwischen der Grundfläche des nach außen hermetisch abgeschlossenen Gehäuses 21 und der 2. Hauptfläche 12.1 der Energieversorgungseinheit 10 hat ein besonders günstiges Grö-ßenverhältnis. Die 2. Hauptfläche 12.1 der Energieversorgungseinheit 10 hat dabei mindestens die 0,7-fache Fläche der Grundfläche des Gehäuses 21 des elektromedizinischen Implantats 20. Günstig ist die 0,7- bis 0,99-fache Fläche, bevorzugt die 0,7- bis 0,95-fache, besonders günstig die 0,8- bis 0,9-fache und besonders bevorzugt die 0,8-fache bis 0,85-fache Größe der Fläche der 2. Hauptfläche 12.1 im Verhältnis zur Grundfläche des Gehäuses 21. Die Höhe darf die 0,7-fache Höhe des nach außen hermetisch abgeschlossenen Gehäuses 21 nicht überschreiten. Günstig ist die Höhe der Energieversorgungseinheit 10 zwischen der 0,4- bis 0,6-fachen, bevorzugt zwischen der 0,5- und 0,6-fachen und besonders bevorzugt zwischen der 0,55- und 0,6-fachen Höhe des Gehäuses 21. Dies ermöglicht eine einfache Positionierung der Energieversorgungseinheit 10 im Gehäuse 21 des elektromedizinischen Implantats 20 und verhindert versehentlichen fehlerhaften oder falschen Einbau der Energieversorgungseinheit 10 in das elektromedizinische Implantat 20. Die Raumausnutzung der Energieversorgungseinheit 10 ist hierbei möglichst hoch, um eine hohe Energiedichte der Energieversorgungseinheit und damit eine lange Lebensdauer des Endgeräts sicherzustellen.

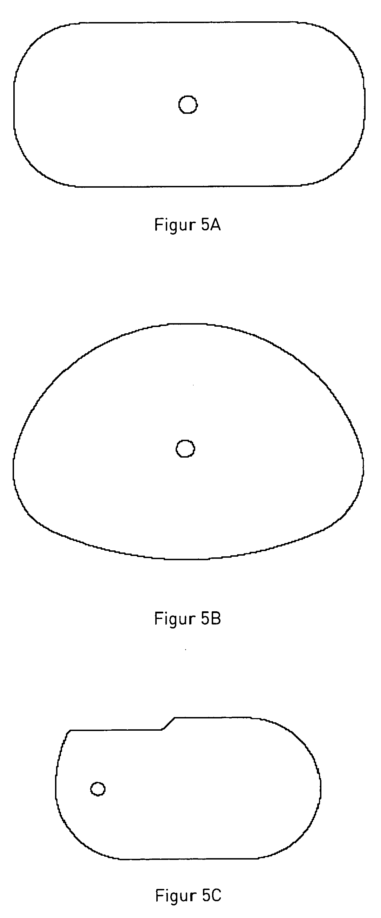

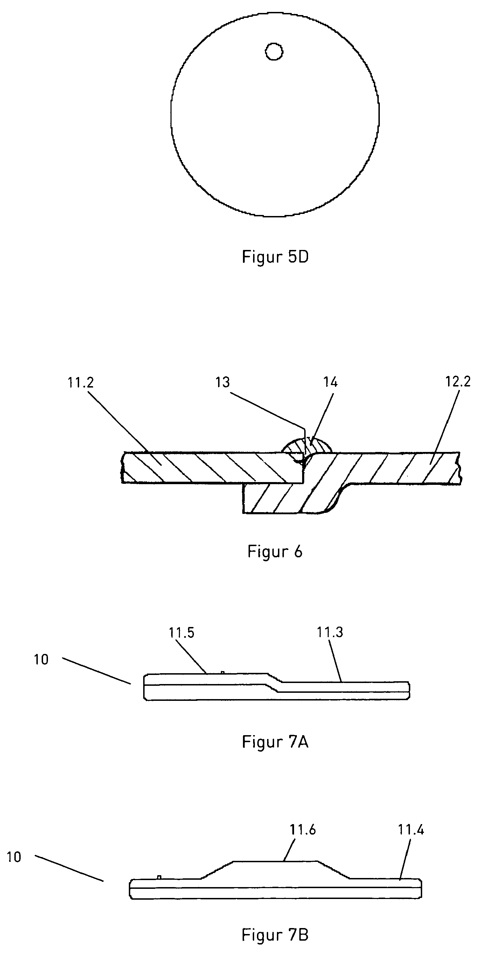

Figur 4 zeigt eine Energieversorgungseinheitseinrichtung 10, die in abgeflachter Bauweise ausgeführt ist. Die Energieversorgungseinheit 10 besteht aus einer ersten Schale 11 mit einer 1. Hauptfläche 11.1 und einer Seitenwand 11.2 und einer zweiten Schale 12 mit einer 2. Hauptfläche 12.1 und einer zweiten Seitenwand 12.2. Beide Schalen 11 und 12 mit ihren Seitenwänden 11.2 und 12.2 sind so geformt, dass beim Zusammenfügen der Seitenwände ein Formschluss entsteht. Dabei bildet sich eine Rille, die das hermetische Verschweißen der beiden Schalen ermöglicht. Die 1. Hauptfläche 11.1 weist eine Glas-Metall-Durchführung 30 auf, die aus einem Durchführungspin 31, einer Buchse 32 und einer Glasisolierung besteht. Die 2. Hauptfläche 12.1 der Energieversorgungseinheit 10 liegt auf der Innenfläche des hermetisch dichten Gehäuses 21 des elektromedizinischen Implantats 20. Die Form der 2. Hauptfläche 12.1 entspricht der Grundfläche des hermetisch dichten Gehäuses 21 des elektromedizinischen Implantats 20. Es sind runde, ovale oder physiologisch geformte Gehäuseformen realisierbar. Mögliche Formen der Hauptflächen sind in den Figuren 5A bis 5D in der Aufsicht dargestellt. Weitere andere Formen sind möglich.

Um auch die Herstellung der Energieversorgungseinheit 10 zu vereinfachen, sind die beiden Seitenwände 11.2 und 12.2 der Schalen 11 und 12 so aufgebaut, dass das Zusammenfügen sehr einfach erfolgt. In Figur 6 ist ein beispielhafter Aufbau der Seitenwände 11.2 und 12.2 kurz vor dem Schweißprozess dargestellt. Die Seitenwände 11.2 und 12.2 bilden dabei ein Formschluss. An der Berührfläche zwischen den Seitenwänden 11.2 und 12.2 bildet sich eine umlaufende Rille 13, an der zum Beispiel mit einem Laserschweiß-Prozess eine hermetisch dichte Schweißnaht 14 aufgebracht werden kann.

Während des Betriebs der Energieversorgungseinheit kann es bei verschiedenen Zuständen geschehen, dass eine Schwellung der Energieversorgungseinheit auftritt. Eine zu starke Schwellung kann die auf der Energieversorgungseinheit fixierte Steuerungseinheit gegen die Innenseite des hermetisch abgeschlossenen Gehäuses des elektromedizinischen Implantats drücken. Dies kann zu Beschädigungen der Steuerungseinheit führen. Deshalb müssen diese Schwellungen gering gehalten werden.

Es hat sich herausgestellt, dass eine Energieversorgungseinheit mit mindestens zwei unterschiedlichen Höhenniveaus vorteilhaft zur Verhinderung dieser Schwellung ist. Wie in Figur 7A und Figur 7B beispielhaft dargestellt besitzt die 1. Hauptfläche 11.1 einer Energieversorgungseinheit 10 ein erstes Niveau 11.3 oder 11.4 und ein zweites Niveau 11.5 oder 11.6. Zwischen dem ersten Niveau 11.3 oder 11.4 und dem jeweiligen zweiten Niveau 11.5 oder 11.6 befindet sich ein Winkel, wobei das Gradmaß dieses Winkels zwischen 30° und 60°, bevorzugt zwischen 40° und 50° und besonders vorteitig von 45° ist. Um eine homogene Raumausnutzung des Innenraums des hermetisch dichten Gehäuses 21 des elektromedizinischen Implantats 20 zu erreichen, muss der Höhenunterschied zwischen dem ersten Niveau 11.3 oder 11.4 und dem zweiten Niveau 11.5 oder 11.6 genauso hoch sein wie die auf dem ersten Niveau 11.3 und 11.4 befestigte Steuerungseinheit 33.

Für ein implantierbares Gerät ist eine dauerhaft elektrisch leitfähige Verbindung zwischen der Energieversorgungseinheit und der elektronischen Steuereinheit von besonderer Bedeutung. Bei sehr vielen Lithium-Batterien ist das Gehäuse elektrisch leitend mit der Anode verbunden und kann von außen direkt am Gehäuse kontaktiert werden. Ein zuverlässiger Kontakt lässt sich bevorzugt durch Schweißen oder Löten herstellen. Nachteilig ist, dass beim direkten Anschweißen oder Anlöten der Steuerungseinheit am Gehäuse der Energieversorgungseinheit Beschädigungen z.B. durch versehentliches Öffnen des Gehäuses (Verlust der hermetischen Abdichtung) oder durch Schädigung von Baugruppen, die durch Wärmeeintrag innerhalb der Energieversorgungseinheit entstehen.

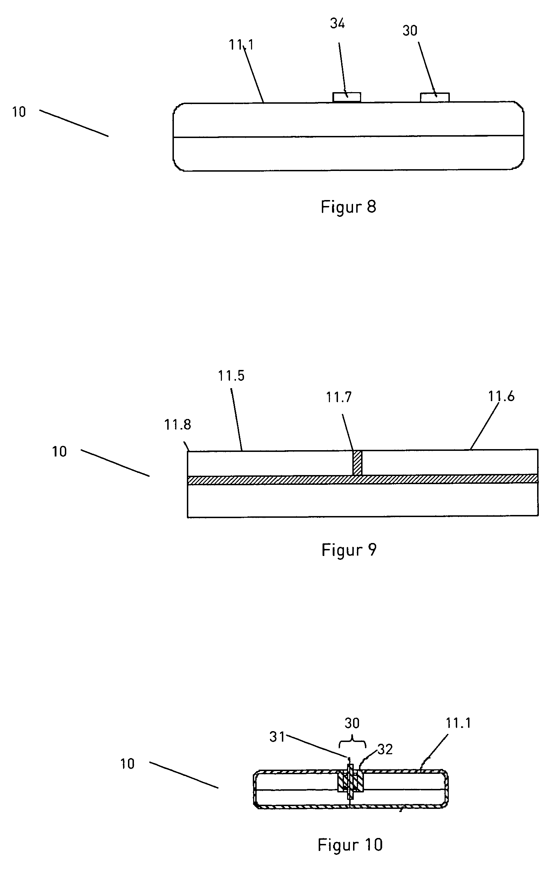

Dieser Nachteil wie zum Beispiel in Figur 8 dargestellt verhindert. Ein oder mehrere Kontaktelemente 34 werden in die 1. Hauptfläche 11.1 der Energieversorgungseinheit 10 integriert. Das Kontaktelement 34 wird vor dem hermetischen Verschluss der Energieversorgungseinheit 10 mit der 1. Hauptfläche 11.1 verbunden. So können eventuelle Beschädigungen der Gehäuseintegrität der Energieversorgungseinheit 10 über einen Lecktest vor dem Verschluss der Energieversorgungseinheit 10 effektiv ausgeschlossen werden. Das Kontaktelement 34 ist so flach wie die Glas-Metall-Durchführung 30 ausgeführt, dass es die Höhe der Energieversorgungseinheit 10 in Verbindung mit der Steuerungseinheit 33 nicht vergrößert. Die Materialstärke des Kontaktstücks 34 ist so groß gewählt, dass Schweißen oder Löten am Kontaktstück 34 möglich ist, ohne das Gehäuse der Energieversorgungseinheit 10 oder die Glas-Metall-Durchführung 30 dabei zu beschädigen. Das Herstellen einer leitfähigen, mechanisch stabilen Verbindung (z.B. durch Schweißen oder Löten) zwischen der Steuerungseinheit 33 des medizinischen elektrischen Gerätes 20 und der 1. Hauptfläche 11.1 der Energieversorgungseinheit 10 wird durch das Kontaktelement 34 erheblich erleichtert und die Gefahr einer Beschädigung der Energieversorgungseinheit 10 durch den Eintrag von Wärme beim Herstellen des elektrischen Kontakts beseitigt.

Während die Anode über die 1. Hauptfläche 11.1 abgegriffen wird, wird die Kathode der Energieversorgungseinheit 10 über einen Pin 31 (in Figur 3) der Glas-Metall-Durchführung 30 abgegriffen. Weiterhin ist es aber auch möglich, sowohl die Anode als auch die Kathode über Glas-Metall-Durchführungen aus der Energieversorgungseinheit 10 herauszuführen. Dies hat den Vorteil, dass die erste Schale 11 mit der 1. Hauptfläche 11.1 der Energieversorgungseinheit 10 nicht elektrisch kontaktiert ist und deshalb keine Isolierung zwischen der Energieversorgungseinheit 10 und dem Gehäuse 21 des elektromedizinischen Implantats 20 vorgesehen werden muss.

Ein weiteres Ausführungsbeispiel ist in Figur 9 zu sehen. Zu sehen ist eine Energieversorgungseinheit 10 mit einer ersten Schale 11 und einer zweiten Schale 12. Die erste Schale 11 hat zwei gegeneinander isolierte Flächen 11.5 und 11.6, die gegeneinander mit einer Isolierung 11.7 isoliert sind. Ebenso kann eine Isolierung 11.8 zwischen der ersten Schale 11 und der zweiten Schale 12 vorgesehen werden. Somit ist es möglich, sowohl die Kathode als auch die Anode über die Hauptfläche 11.1 -die in diesem Fall aus den Flächen 11.5, 11.6 und der Isolierung 11.7 besteht- zweipolig zu kontaktieren. Dies hat Vorteile bei der Vereinfachung der elektrischen Verbindung zwischen der Energieversorgungseinheit 10 und der elektrischen Steuerungseinheit 33 des elektromedizinischen Implantats 20. Bei dieser Ausführungsvariante kommt auch das in Figur 8 dargestellte Kontaktelement 34 zur Anwendung, dann jedoch offensichtlich für jede Teilfläche 11.5 und11.6 jeweils mindestens ein Kontaktelement.

Figur 10 zeigt die Integration einer Glas-Metall-Durchführung 30 in die 1. Hauptfläche 11.1 der Energieversorgungseinheit 10. An dem Pin 31 der Glas-Metall Durchführung wird ein Pol der Energieversorgungseinheit 10 -isoliert von dem Metall der 1. Hauptfläche 11.1- aus dem Inneren der Energieversorgungseinheit 10 nach außen geführt. Die Glas-Metall-Durchführung 30 gewährleistet den hermetischen Verschluss der Energieversorgungseinheit 10. Besonders vorteilhaft ist der besondere Einbau der Glas-Metall-Durchführung 30: Die Buchse 32 der Glas-Metall-Durchführung 30 ist außenseitig bündig in die 1. Hauptfläche 11.1 eingeschweißt. Dies gewährleistet, dass die Buchse 32 der Durchführung 30 nicht über die äußere Fläche der 1. Hauptfläche 11.1 übersteht. Der Pin 31 der Glas-Metall-Durchführung 30 ist so kurz ausgelegt, dass er gerade so weit über die 1. Hauptfläche 11.1 der Energieversorgungseinheit 10 herausragt, dass eine elektrisch leitende Kontaktierung der Steuerungseinheit 33 ermöglicht wird. Der Vorteil dieser Konstruktion ist, dass hiermit eine besonders flache, Raum sparende Anordnung der Energieversorgungseinheit 10 und der Steuerungseinheit 33 im Gehäuse 21 des implantierbaren Geräts 20 ermöglicht wird.

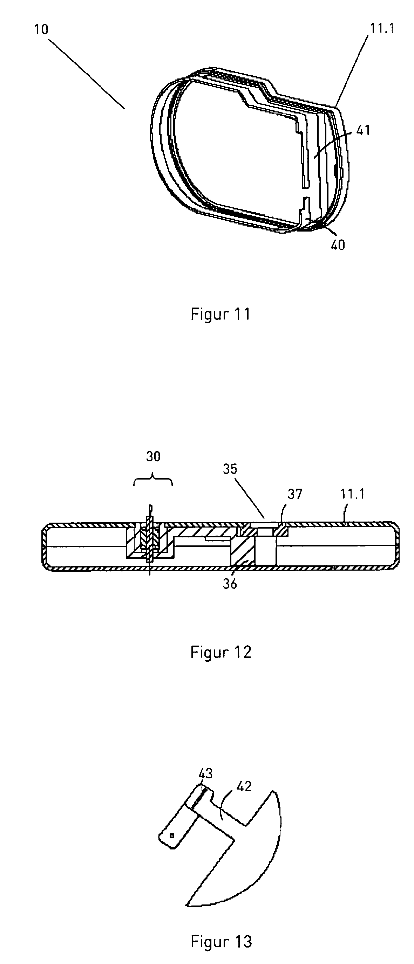

In Figur 11 ist eine Lösung auch für komplexe Geometrien dargestellt, den Abstand der Elektroden zum Separator und damit den Abstand der Elektroden zueinander möglichst präzise einzuhalten. Diese Anforderung wurde durch die Verwendung eines speziellen Halterings 40 in einer Energieversorgungseinheit erfüllt, welcher den Separator fest und homogen auf die Elektrode der Energieversorgungseinheit aufspannt. Dieser Haltering 40 ist vorzugsweise aus einem speziellen Kunststoff gefertigt, der gegenüber anderen Bestandteilen der Energieversorgungseinheit inert ist (z.B. gegenüber dem Elektrolyten) und gleichzeitig ein elektrischer Isolator ist. Bevorzugt geeignet sind Polyethylen und Polypropylen sowie polyhalogenierte Olefine, welche sich thermoplastisch formen lassen (z.B. TEFLON®, HALAR®, KYNAR® oder SOLEF®]. Wenn der Separator elektrisch isolierend wirkt, kann der Haltering 40 auch unterhalb des Separators geführt werden. In diesem Fall kann der Haltering 40 auch aus einem gegenüber anderen Bestandteilen der Energieversorgungseinheit inertem Metall, z.B. Edelstahl, ausgeführt sein. Der genannte Haltering 40 ist mit einer Öffnung 41 versehen, durch die der Ableiter der Kathode geführt werden kann. Dieses besondere konstruktive Merkmal ermöglicht eine besonders einfache und Platz sparende Anbindung der Kathode an die auf 1. Hauptfläche 11.1 der Energieversorgungseinheit 10 befindliche Glas-Metall-Durchführung 30, da der Ableiter der Kathode nicht gebogen oder senkrecht zur Energieversorgungseinheitshauptachse zur Glas-MetaLL-Durchführung 30 geführt werden muss.

Figur 12 zeigt, dass in die 1. Hauptfläche 11.1 der Energieversorgungseinheit 10 eine Befüllöffnung 35 integriert ist. Durch eine Befüllöffnung 35 wird ein flüssiger oder gelförmiger Elektrolyt in die Energieversorgungseinheit 10 eingefüllt. Ein Vorteil ist die besondere Konstruktion der Befüllöffnung 35. Unter der Befüllöffnung 35 befindet sich ein spezielles Druckstück 36, welches zusammen mit dem Haltering 40 die Stabilität der inneren Konstruktion der Zelle sicherstellt. Dieses Druckstück 36 ist weiterhin so gestaltet, dass es das Einschweißen einer versenkten Kappe 37 ermöglicht, die die Energieversorgungseinheit nach dem BefüLLvorgang hermetisch verschließt. Die versenkte Kappe 37 baut nicht auf der 1. Hauptfläche 11.1 der Energieversorgungseinheit 10 auf und stellt die flache, Raum sparende Montage der Steuerungseinheit 33 auf der Energieversorgungseinheit 10 sicher.

Beim Befüllvorgang wird die Energieversorgungseinheit evakuiert. Der Elektrolyt wird durch den Unterdruck in der Zelle eingesaugt (vacuum filling). Bei flachen Energieversorgungseinheiten ist dieses Verfahren besonders schwierig, weil sich die Hauptseiten der Energieversorgungseinheit durch den Außendruck verformen können. Ein weiteres zu lösendes technisches Problem ist die Platzierung der Befüllstutzens. Dieser muss mit einer Dichtung über die Befüllöffnung gepresst werden, um das Eindringen von Luft beim Evakuieren effektiv zu verhindern. Hierbei besteht das Risiko, dass sich das Gehäuse der Energieversorgungseinheit durch das Aufpressen des Befüllstutzens zu verformt. Auch beim Eindrücken der Verschlusskappe wird der Bereich der Befüllöffnung der Energieversorgungseinheit mechanisch belastet.

Das Druckstück 36. stabilisiert die Geometrie der Energieversorgungseinheit 10 -insbesondere im Bereich der Befüllöffnung 35. Hierdurch kann der Befüllstutzen mit größerem Druck (bessere Abdichtung) aufgepresst werden. Gleichzeitig wird eine Verformung des Gehäuses der Energieversorgungseinheit 10 während des Evakuierens und eine Verformung beim Aufpressen der Verschlusskappe 37 verhindert. Durch die spezielle Form des Druckstücks 36 wird zugleich die mechanisch empfindliche Glas-Metall-Durchführung 30 umschlossen und so zusätzlich vor mechanischer Beschädigung geschützt. Dies ermöglicht auch die Platzierung der Befüllöffnung 35 in unmittelbarer Nähe der Glas-Metall-Durchführung 30.

Auch der Haltering 40 stabilisiert die Geometrie der Energieversorgungseinheit 10, da er den Raum zwischen der Innenseite der 1. und 2. Hauptflächen 11.1 und 12.1 und den Elektroden füllt. Die inneren Baugruppen der Energieversorgungseinheit 10 werden auf diese Weise mechanisch fixiert und können nicht verrutschen. Die vollständige Ausfüllung des Raums außerhalb der Elektroden stabilisiert die Energieversorgungseinheit 10 zusätzlich, da kein Freiraum innerhalb zur Verfügung steht, in den sich das Gehäuse z.B. beim Evakuieren oder Befüllen verformen kann.

An den Pin 31 der Glas-Metall-Durchführung 30 ist innenseitig ein Kontaktblech 42 angeschweißt (Fig. 13). Dieses Kontaktblech 42 ist mit einem Spalt 43 versehen. Das aus der Elektrode herausgeführte Ableitungsgitter wird auf den Spalt 43 aufgelegt und durch den Spalt 43 mit der metallischen Unterlage verschweißt. Durch diese Konstruktion wird der Schweißvorgang vereinfacht, da die Materialien nicht auf Stoß miteinander verschweißt werden müssen. Der Schweißvorgang wird auch dadurch vereinfacht, dass alle zu verschweißenden Materialien in der Hauptachse der Energieversorgungseinheit angeordnet sind. Die Konstruktion von Energieversorgungseinheiten mit komplexer Elektrodengeometrie wird erheblich vereinfacht.

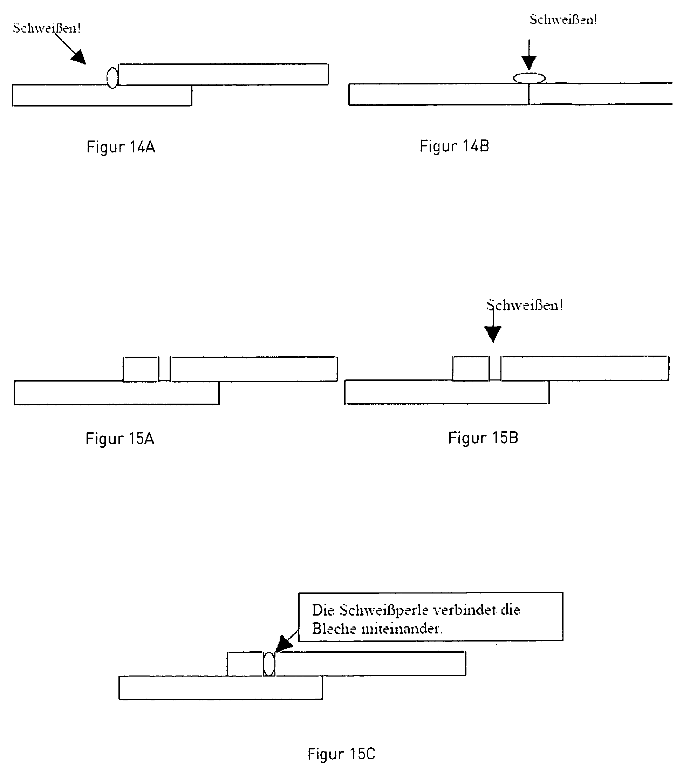

Das Ableitungsgitter der Elektrode wird auf dem Spalt aufgelegt (Figur 15A). Das Ableitungsgitter der Elektrode und das Ableitungsblech werden durch den Spalt miteinander in einer Raumebene verbunden (Figur 15B/15C).

Die Alternative zu diesem Verfahren ist das Verschweißen auf Stoß [Figur 14B] oder über die Kante (Figur 14A).

Die Verfahren aus Figur 14A und 14B sind wesentlich komplizierter, da für 14A eine präzise, schräg ausgeführte Laserschweißung und für 14B eine sehr präzise Positionierung der Bauteile erforderlich ist. Der Vorteil des Verfahrens ist die einfache Positionierung der Bauteile und die Möglichkeit direkt von oben zu verschweißen.

Die Glas-Metall-Durchführung 30, die Befüllöffnung 35 mit Verschlusskappe 37 und die Kontaktelemente 34 werden so auf der 1. Hauptfläche 11.1 positioniert, dass eine ausreichend große freie Fläche zur Unterbringung der Steuerungseinheit 33 zur Verfügung steht, aber gleichzeitig ein direkter Anschluss der Steuerungseinheit 33 an die Pole gewährleistet ist. Durch die Positionierung der Kontaktelemente 34 und der Glas-Metall-Durchführung 30 auf der 1. Hauptfläche 11.1 und deren flache Konstruktion wird der Aufbau des elektrochemischen lmplantats in einer Ebene ermöglicht. Diese Konstruktion stellt eine starke Vereinfachung der Kontaktierung dar.

## Patentansprüche

1. Elektromedizinisches Implantat (20) bestehend aus:
- Einem nach außen hermetisch abgeschlossenen Gehäuse (21),
- Einer Energieversorgungseinheit (10) mit einer ersten Schale (11) mit einer 1. elektrisch leitfähigen Hauptfläche (11.1) und einer 1. Seitenwand (11.2), und einer zweiten Schale (12) mit einer 2. Hauptfläche (12.1) und einer 2. Seitenwand (12.2), die in das nach außen hermetisch abgeschlossene Gehäuse (21) eingebettet ist,
- Einer mit der Energieversorgungseinheit (10) elektrisch verbundenen elektrischen Steuerungseinheit (33)
**dadurch gekennzeichnet, dass**
- Die elektrische Steuerungseinheit (33) über die 1. Hauptfläche (11.1) der Energieversorgungseinheit (10) zweipolig elektrisch verbunden ist,
- Die 2. Hauptfläche (12.1) der Energieversorgungseinheit (10) mindestens die 0,7-fache Fläche der Grundfläche des Gehäuses (21) beträgt und
- Die Höhe der Energieversorgungseinheit (10) höchstens die 0,5-fache Höhe des nach außen hermetisch abgeschlossenen Gehäuses beträgt.

2. Elektromedizinisches Implantat (20) nach Anspruch 1 **dadurch gekennzeichnet, dass** die erste und zweite Schale (11 und 12) der Energieversorgungseinheit [10] mit ihren beiden Seitenwänden (11.2 und 12.2) so geformt sind, dass beim Zusammenfügen der Schalen ein Formschluss entsteht, der ein leichtes hermetisches Verschweißen ermöglicht.

3. Elektromedizinisches Implantat (20) nach einem der vorhergehenden Ansprüche, **dadurch gekennzeichnet, dass** ein erster Pol der elektrischen Verbindung über eine Glas-Metall-Durchführung (30) auf der 1. Hauptfläche (11.1) der Energieversorgungseinheit [10] ist und dass dieser erste Pol vorzugsweise kathodisch ist.

4. Elektromedizinisches Implantat (20) nach Anspruch 3, **dadurch gekennzeichnet, dass** die Glas-Metall-Durchführung (30) in der 1. Hauptfläche (11.1) der Energieversorgungseinheit (10) eine Buchse (32) hat.

5. Elektromedizinisches Implantat (20) nach Anspruch 1 oder 2, **dadurch gekennzeichnet, dass** ein zweiter Pol der elektrischen Verbindung die 1. Hauptfläche (11.1) der Energieversorgungseinheit (10) bildet und dass dieser zweite Pol vorzugsweise anodisch ist.

6. Elektromedizinisches Implantat (20) nach Anspruch 1, 2 oder 5, **dadurch gekennzeichnet, dass** die elektrische Verbindung des zweiten Pols über Kontaktelemente (34) auf der elektrisch leitfähigen 1. Hauptfläche (11.1) der Energieversorgungseinheit (10) hergestellt wird.

7. Elektromedizinisches Implantat (20) nach einem der vorhergehenden Ansprüche, **dadurch gekennzeichnet, dass** die 1. Hauptfläche (11.1) der Energieversorgungseinheit (10) mindestens zwei unterschiedlichen Höhenniveaus (11.3, 11.4, 11.5, 11.6) aufweist, wobei der Höhenunterschied zwischen zwei Höhenniveaus gleich der Höhe der auf dem unteren Niveau [11.3, 11.4] befestigten Steuerungseinheit [33] ist.

8. Elektromedizinisches Implantat [20] nach Anspruch 7, **dadurch gekennzeichnet, dass** die Verbindung zwischen den mindestens zwei unterschiedlichen Höhenniveaus (11.3, 11.4, 11.5, 11.6) einen Winkel ausweist, der zwischen 30° und 60°, vorzugsweise zwischen 40° und 50° im Gradmaß Liegt, vorzugsweise jedoch 45° im Gradmaß beträgt.

9. Elektromedizinisches Implantat [20] nach einem der vorhergehenden Ansprüche, **dadurch gekennzeichnet, dass** die 2. Hauptfläche (12.1) der Energieversorgungseinheit (10) eben ist und in ihrer Form dem nach außen hermetisch abgeschlossenen Gehäuse (21) entspricht.

10. Elektromedizinisches Implantat (20) nach einem der vorhergehenden Ansprüche, **dadurch gekennzeichnet, dass** die 2. Hauptfläche (12.1) der Energieversorgungseinheit (10) die 0,7- bis 0,99-fache, vorzugsweise die 0,7-bis 0,95-fache Grundfläche des Gehäuses (21) hat, besonders günstig jedoch die 0,8- bis 0,9-fache und besonders bevorzugt die 0,8- bis 0,85-fache Grundfläche des Gehäuses (21) des elektromedizinischen Implantats (20) aufweist.
